Europäisches Patentamt

European Patent Office

Office européen des brevets

(19)

(11) **EP 0 714 700 B1**

(12) **EUROPÄISCHE PATENTSCHRIFT**

(45) Veröffentlichungstag und Bekanntmachung des Hinweises auf die Patenterteilung:
**19.08.1998 Patentblatt 1998/34**

(51) Int. Cl.$^6$: **B01J 37/02**

(21) Anmeldenummer: **95118264.1**

(22) Anmeldetag: **21.11.1995**

(54) **Verfahren zur Herstellung eines Katalysators, bestehend aus einem Trägerkörper und einer auf der Oberfläche des Trägerkörpers aufgebrachten katalytisch aktiven Oxidmasse**

Process of manufacturing of a catalyst consisting of a carrier and a catalytic active mass of oxide deposited on the surface of the carrier

Procédé de fabrication d'un catalyseur constitué par un support et une masse d'un oxyde catalytiquement actif déposée sur la suface du support

(84) Benannte Vertragsstaaten:
**BE DE DK ES FR GB IT NL**

(30) Priorität: **29.11.1994 DE 4442346**

(43) Veröffentlichungstag der Anmeldung:
**05.06.1996 Patentblatt 1996/23**

(73) Patentinhaber:
**BASF AKTIENGESELLSCHAFT**
**67056 Ludwigshafen (DE)**

(72) Erfinder:
- **Tenten, Andreas, Dr.**
  **D-67487 Maikammer (DE)**
- **Weidlich, Peter, Dr.**
  **D-68159 Mannheim (DE)**
- **Linden, Gerd, Dr.**
  **D-69117 Heidelberg (DE)**

(56) Entgegenhaltungen:
**EP-A- 0 017 000          WO-A-95/26820**
**US-A- 4 034 060**

**Beschreibung**

Vorliegende Erfindung betrifft ein Verfahren zur Herstellung eines Katalysators, der aus einem Trägerkörper und einer auf der Oberfläche des Trägerkörpers aufgebrachten katalytisch aktiven Oxidmasse besteht, bei dem man den Trägerkörper zunächst mit einer Haftflüssigkeit (einem flüssigen Bindemittel) befeuchtet, danach durch Inkontaktbringen mit trockener, feinteiliger, aktiver Oxidmasse an der Oberfläche des befeuchteten Trägerkörpers eine Schicht (Schale) aktiver Oxidmasse anheftet (anbringt) und anschließend die Haftflüssigkeit aus dem mit aktiver Oxidmasse beschichteten befeuchteten Trägerkörper entfernt.

Ferner betrifft vorliegende Erfindung Katalysatoren, die aus einem Trägerkörper und einer auf der Oberfläche des Trägerkörpers aufgebrachten katalytisch aktiven Oxidmasse bestehen und als Schalenkatalysatoren bezeichnet werden, sowie die Verwendung solcher Schalenkatalysatoren.

Es ist allgemein bekannt, daß oxidative chemische Umsetzungen häufig in vorteilhafter Weise in der Gasphase an katalytisch aktiven Oxiden durchgeführt werden können. So betrifft die DE-A 23 51 151 die katalytische Oxidation, Ammoxidation sowie die oxidative Dehydrierung von 3 bis 5 C-Atome aufweisenden Olefinen an katalytisch aktiven Oxidmassen in der Gasphase. Beispielhafte Ausführungsformen bilden die Umsetzung von Butadien zu Maleinsäureanhydrid, von Propen zu Acrolein, von Acrolein zu Acrylsäure, von Propen zu Acrylnitril sowie von 2-Buten zu Butadien. Die DE-A 16 42 921 und die DE-A 21 06 796 lehren die katalytische Gasphasenoxidation von aromatischen und ungesättigten Kohlenwasserstoffen, Naphthalin, o-Xylol, Benzol oder n-Buten zu Carbonsäuren oder deren Anhydriden. Beispielhafte Ausführungsformen bilden die Umsetzung von o-Xylol zu Phthalsäureanhydrid sowie von Butadien zu Maleinsäureanhydrid. Aus der DE-A 25 26 238 ist bekannt, Acrylsäure oder Methacrylsäure durch katalytische Gasphasenoxidation von Acrolein oder Methacrolein an katalytisch aktiven Oxidmassen zu erzeugen. Die DE-A 20 25 430 betrifft die katalytische Gasphasenoxidation von Indanen zu z.B. Anthrachinon. Die katalytisch aktive Oxidmasse kann neben Sauerstoff lediglich ein anderes Element oder mehr als ein anderes Element (Multielementoxidmassen) enthalten.

Besonders häufig kommen katalytisch aktive Oxidmassen zur Anwendung, die mehr als ein metallisches, insbesondere übergangsmetallisches, Element umfassen. In diesem Fall spricht man von Multimetalloxidmassen. Üblicherweise sind Multielementoxidmassen keine einfachen physikalischen Gemische von Oxiden der elementaren Konstituenten, sondern heterogene Gemische von komplexen Polyverbindungen dieser Elemente.

In der Regel erfolgt die großtechnische Realisierung solcher katalytischer Gasphasenoxidationen in Festbettreaktoren. D.h. das Reaktionsgasgemisch durchströmt eine ruhende Katalysatorschüttung und die oxidative chemische Umsetzung erfolgt während der Verweilzeit in der selbigen.

Die meisten katalytischen Gasphasenoxidationen verlaufen stark exotherm, weshalb man sie anwendungstechnisch zweckmäßig in Vielkontaktrohr-Festbettreaktoren durchführt. Die Kontaktrohrlänge erstreckt sich im Normalfall auf wenige Meter und der Kontaktrohrinnendurchmesser beläuft sich regelmäßig auf wenige Zentimeter. Die Kontaktrohre umströmende Wärmeaustauschmittel führen die Prozeßwärme ab (vgl. z.B. die DE-A 44 31 957 und die DE-A 44 31 949).

Festbettschüttungen aus feinteiliger, pulverförmiger, katalytisch aktiver Oxidmasse sind zur Durchführung katalytischer Gasphasenoxidationen wenig geeignet, da sie wirtschaftlichen Belastungen mit Ausgangsreaktionsgasgemisch normalerweise nicht ohne hydraulische Förderung standzuhalten vermögen.

D.h. üblicherweise werden aus der katalytisch aktiven Oxidmasse Formkörper gebildet, deren Längstausdehnung, dem Kontaktrohrinnendurchmesser angemessen, in der Regel einige Millimeter beträgt.

Als bevorzugte Geometrie solcher Formkörper empfiehlt das US-A 4 366 093 generell Hohlzylinder (Ringe). Höhe und Außendurchmesser sollen 3 bis 6 mm und die Wandstärke 1 bis 1,5 mm betragen. Als Formgebungsmethode zieht das US-A 4 366 093 lediglich das Tablettieren oder das Extrudieren zu Vollkatalysatoren (der gesamte Hohlzylinder besteht aus katalytisch aktiver Masse, die allenfalls mittels feinteiligem, inerten Material verdünnt ist) oder das Imprägnieren von Trägerringen zu Trägerkatalysatoren in Betracht. Nachteilig an ringförmigen Vollkatalysatoren einer ≤ 1,5 mm betragenden Wandstärke ist deren beim Einfüllen in die Kontaktrohre nicht voll befriedigende mechanische Stabilität. Nachteilig an Trägerkatalysatoren ist, daß sie auf solche oxidischen Aktivmassen beschränkt sind, die aus Lösungen heraus gebildet werden können. Außerdem bedingt eine einmalige Tränkung lediglich eine geringe Aufnahme an Aktivmasse.

Das US-A 4 438 217 und das US-A 4 522 671 empfehlen zur gasphasenkatalytisch oxidativen Herstellung von Acrolein bzw. Methacrolein Vollkatalysator-Ringe eines Außendurchmessers von 3 bis 10 mm, eines Innendurchmessers, der das 0,1 bis 0,7fache des Außendurchmessers und einer Höhe, die das 0,5 bis 2fache des Außendurchmessers beträgt, auf der Basis von Molybdän als Hauptbestandteil enthaltenden Mulitmetalloxiden. Als Untergrenze für die Wandstärke wird mit Blick auf die erforderliche mechanische Stabilität 1 mm als gerade noch möglich erachtet. Nachteilig an größeren Wandstärken ist jedoch, daß mit ihnen eine Verlängerung des Diffusionsweges aus der Reaktionszone heraus einhergeht, was unerwünschte Folgereaktionen fördert und damit die Zielproduktselektivität mindert.

Das US-A 4 537 874 empfiehlt zur gasphasenkatalytisch oxidativen Herstellung α,β-monoethylenisch ungesättigter

2

EP 0 714 700 B1

Aldehyde ebenfalls Katalysatorschüttungen aus Vollkatalysator-Ringen auf der Basis von Molybdän als Hauptbestandteil enthaltenden Multimetalloxiden. Die Wandstärke der Hohlzylinder beträgt in allen Ausführungsbeispielen 2 mm.

Eine Auflösung des bei Vollkatalysator-Ringen bestehenden Widerspruchs zwischen erforderlicher mechanischer Stabilität (zunehmender Wandstärke) einerseits und Begrenzung des Diffusionsweges aus der Reaktionszone heraus (abnehmende Wandstärke) andererseits unter Aufrechterhaltung der ansonsten besonders vorteilhaften Ringgeometrie eröffnen ringförmige Schalenkatalysatoren. Die mechanische Stabilität wird durch den ringförmigen Träger gewährleistet und auf der Ringoberfläche kann die katalytisch aktive Oxidmasse in der gewünschten Schichtdicke aufgebracht werden.

Problematisch bei Schalenkatalysatoren ist jedoch ganz generell deren Herstellung im technischen Maßstab, d.h. sie im technischen Maßstab so herzustellen, daß

- sie die im Hinblick auf die Katalysatoraktivität geforderte Schichtdicke aufweisen,

- die katalytisch aktive Schale in der geforderten Dicke in befriedigender Weise auf der Oberfläche des Trägerkörpers haftet,

- die Schalendicke, über die Oberfläche eines Trägerkörpers betrachtet, möglichst geringe Schwankungen aufweist,

- die Schalendicke, über die Oberfläche verschiedener Trägerkörper betrachtet, möglichst geringe Schwankungen aufweist,

- die Größe der spezifischen, auf die Masseneinheit der Aktivmasse bezogenen, katalytisch aktiven Oberfläche befriedigt und

- die Produktionsleistung des Herstellverfahrens befriedigt.

Dies gilt insbesondere im Fall von hohlzylindrischen Trägerkörpern, deren Rollverhalten im Unterschied zu Trägerkugeln eine Vorzugsrichtung aufweist und dürfte ursächlich dafür sein, daß die verfahren zur Herstellung von katalytisch aktive Oxidmassen aufweisenden Schalenkatalysatoren im Stand der Technik im wesentlichen auf kugelförmige Schalenkatalysatoren beschränkt sind.

Aus der DE-A 20 25 430 ist bekannt, daß man Schalenkatalysatoren auf der Basis katalytisch aktiver Oxidmassen dadurch herstellen kann, daß man das katalytisch aktive Material mit Hilfe des Plasmaspritz-oder Flammspritzverfahrens auf den Trägerkörper aufbringt. Nachteilig hinsichtlich der Anwendbarkeit dieses Verfahrens ist, daß die Schmelzbarkeit mindestens einer Hauptkomponente bei der Arbeitstemperatur des Flammspritz- oder des Plasmabrenners gegeben sein muß. Ein weiterer Nachteil dieses Verfahrens besteht darin, daß die Größe der spezifischen katalytisch aktiven Oberfläche in der Regel nicht zu befriedigen vermag. Bei allen beispielhaften Ausführungsformen der DE-A 20 25 430 handelt es sich um kugelförmige Schalenkatalysatoren. Als Vergleichsbeispiel enthält die DE-A 20 25 430 ein Verfahren zur Herstellung eines kugelförmigen Schalenkatalysators, bei dem eine Oxalsäure und die katalytisch aktive Oxidmasse gelöst enthaltende wäßrige Lösung auf heiße Trägerkugeln aufgesprüht wird. Nachteilig an dieser verfahrensweise ist, daß sie nur bei in Wasser löslichen katalytisch aktiven Oxidmassen angewendet werden kann. Außerdem führt sie zu unregelmäßiger Schalendicke sowie nicht befriedigender Schalenhaftung infolge der schlagartigen Verdampfung des Lösungsmittels an der Oberfläche der heißen Trägerkugel. Auch bedingt diese Verfahrensweise nicht unerhebliche Aktivmassenverluste.

Die DE-A 16 42 921 betrifft die Herstellung kugelförmiger oxidischer Schalenkatalysatoren durch Aufsprühen einer die oxidische Aktivmasse in gelöster oder suspendierter Form enthaltenden Flüssigkeit auf heiße kugelförmige Trägerkörper. Als Lösungsmittel bzw. Suspendiermedium empfiehlt die DE-A 16 42 921 Wasser oder ein organisches Lösungsmittel wie Alkohol bzw. Formamid. Nachteilig ist auch hier u.a., daß das Wasser bzw. Lösungsmittel praktisch auf einen Schlag verdampft, sobald die aufgesprühte Masse mit dem heißen Träger in Berührung kommt, was die Haftfestigkeit der Schale mindert.

Die Lehre der DE-A 25 10 994 entspricht im wesentlichen der Lehrer der DE-A 16 42 921 mit dem Unterschied, daß sie auch ringförmige Träger einschließt. Sie ist außerdem auf solche katalytisch aktiven Oxidmassen beschränkt, die im wesentlichen aus einem Vanadin-Titan-Mischoxid bestehen.

Aus der DE-A 21 06 796 ist die Herstellung von Schalenkatalysatoren bekannt, indem man wäßrige Suspensionen des katalytisch wirksamen oxidischen Materials auf die bewegten Trägerkörper sprüht. Diese verfahrensweise weist dieselben Nachteile auf, wie bereits für das Aufsprühen von die oxidische Aktivmasse gelöst enthaltenden, wäßrigen Lösungen beschrieben. Dies gilt insbesondere für das Auf sprühen auf erhitzte Trägerkörper. Diesen Nachteilen vermag auch die Empfehlung der Mitverwendung einer wäßrigen Polymerisatdispersion als Bindemittel nicht abzuhelfen, vielmehr erschwert das Beisein einer Polymerisatdispersion den Beschichtungsvorgang durch schwer kontrollierbare Filmbildungsprozesse. Die DE-AS 21 06 796 nennt zwar auch Zylinder als brauchbare Trägerkörper, die beispielhaften Ausführungsformen umfassen jedoch kein entsprechendes Ausführungsbeispiel.

Die DE-A 26 26 887 versucht die Nachteile der DE-A 21 06 796 dadurch zu mindern, daß das Aufsprühen der wäßrigen Suspension auf lediglich eine Temperatur von 25 bis 80°C aufweisende Trägerkugeln erfolgt. Gemäß der DE-A 29 09 671, Seite 5, Zeile 10, kann es bei dieser verfahrensweise jedoch zu Verklebungen der besprühten Trägerkörper kommen. Zur Erhöhung der Haftfestigkeit der oxidischen katalytisch aktiven Schale auf der Oberfläche des Trägerkörpers empfiehlt die DE-A 26 26 887 das Einarbeiten anorganischer Hydroxysalze in die aufzusprühende wäßrige Suspension, die in wäßriger Lösung zu Hydroxiden hydrolisieren und nach Fertigstellung des Schalenkatalysators katalytisch indifferente Bestandteile der katalytisch aktiven Oxidmasse bilden. Nachteilig an dieser Maßnahme ist jedoch, daß sie eine verdünnung der oxidischen Aktivmasse bedingt. Als mögliche Trägerkörper nennt die DE-A 26 26 887 zwar auch Ringe und Zylinder, die Gesamtheit der Ausführungsbeispiele ist jedoch auf kugelförmige Schalenkatalysatoren beschränkt.

Die Lehre der DE-A 29 09 670 entspricht im wesentlichen derjenigen der DE-A 26 26 887. Gemäß Beschreibung der DE-A 29 09 670 können als Suspendiermedium auch Gemische aus Wasser und Alkohol verwendet werden. Nach Beendigung des Aufsprühens der Suspension der katalytisch aktiven Oxidmasse wird durch Überleiten von heißer Luft der Feuchtigkeitsgehalt beseitigt. Die beispielhaften Ausführungsformen der DE-A 29 09 670 umfassen auch ringförmige Schalenkatalysatoren. In allen beispielhaften Ausführungsformen wird jedoch lediglich Wasser als Suspendiermedium verwendet. Nachteilig an der Verfahrensweise der DE-A 29 09 670 ist, wie bereits mit Bezug auf die DE-A 26 26 887 erwähnt, die Neigung der besprühten Formkörper zum Agglomerieren. Ferner vermag bei den ringförmigen Schalenkatalysatoren in der Regel die resultierende spezifische, katalytisch aktive Oberfläche der Oxidmassenschale nicht zu befriedigen.

Die GB-1 331 423 betrifft ein Verfahren zur Herstellung von kugelförmigen oxidischen Schalenkatalysatoren, das dadurch gekennzeichnet ist, daß man aus Katalysatorvorläufern und einer organischen Hilfssubstanz, deren Siedepunkt bei Normaldruck wenigsten 150°C beträgt und die in Wasser löslich ist, eine wäßrige Suspension oder Lösung bildet, selbige mit den Trägerkörpern versetzt und unter gelegentlichem Umrühren die flüssigen Bestandteile durch verdampfen entfernt. Anschließend werden die so erhaltenen beschichteten Trägerkörper calciniert und die Katalysatorvorläuferschicht in aktives Oxid umgewandelt. Nachteilig an dieser verfahrensweise ist, daß die resultierenden Schalenkatalysatoren eine relativ unregelmäßige Schalendicke aufweisen. Ferner vermag die Haftung der Schale auf der Trägerkörperoberfläche nicht zu befriedigen, da die Calcination der Katalysatorvorläufermasse in der Regel in unkontrollierter Weise gasförmige Verbindungen freisetzt, die eine Auflockerung des Gefüges bewirken.

Die EP-A 286 448 und die EP-A 37 492 empfehlen die Herstellung von Schalenkatalysatoren nach den bereits beschriebenen Sprühverfahren, bzw. nach dem Verfahren der GB-1 331 423, mit den bereits benannten Nachteilen.

Das EP-B 293 859 offenbart ein Verfahren zur Herstellung von kugelförmigen Schalenkatalysatoren durch Anwendung einer zentrifugalen Strömungs-Beschichtungsvorrichtung. Diese Verfahrensweise führt zu besonders gleichmäßig gearbeiteten Schalendicken, sowohl über die einzelne Kugeloberfläche als auch über verschiedene Kugeloberflächen betrachtet. Nachteilig an der Verfahrensweise der EP-B 293 859 ist jedoch, daß die EP-B 293 859 empfiehlt, die Trägerkugeln nicht unmittelbar mit der katalytisch aktiven Oxidmasse sondern mit einer Vorläufermasse derselben zu beschichten. Letztere wird durch anschließendes Brennen (Calcinieren) bei erhöhter Temperatur (einige hundert Grad Celsius) in erstere überführt. In der Regel gehen mit der Calcinierung spontan, d.h. mehr oder weniger unkontrolliert verlaufende thermische Zersetzungen von in der Vorläufermasse enthaltenen Komponenten zu gasförmigen Produkten einher, die zwar einerseits die Ausbildung spezieller Porenverteilungen sowie einer erhöhten spezifischen katalytisch aktiven Oberfläche (bis zu 15 $m^2$/g) bedingen, andererseits die Haftung der katalytisch aktiven Schale auf der Oberfläche des Trägerkörpers mindern. Eine Calcination nach Beschichten ist auch insofern von Nachteil, als beim Calcinieren Fehlchargen erzeugt werden können (z.B. bei fehlerhafter Calcinationsatmosphäre). Eine Aufarbeitung derselben ist bei bereits durchgeführter Beschichtung wesentlich komplizierter. Als Bindemittel nennt das EP-B 293 859 Wasser, Alkohol und Aceton neben Ammoniumnitrat, Graphit und Stärke.

Aus der DE-A 25 26 238, der US-3 956 377 und der DE-A 235 151 ist ein Verfahren zur Herstellung kugelförmiger Oxid-Schalenkatalysatoren bekannt, bei dem die Trägerkugeln zunächst mit Wasser oder einer anderen Flüssigkeit wie Petrolether als Bindemittel befeuchtet werden. Anschließend wird die katalytisch aktive Oxidmasse dadurch auf das mit Bindemittel angefeuchtete Trägermaterial aufgebracht, daß man das feuchte Trägermaterial in der pulverförmigen katalytisch aktiven Oxidmasse wälzt. Nachteilig an dieser Verfahrensweise ist, daß die erreichbare Schalendicke durch das Bindemittelaufnahmevermögen des Trägers beschränkt wird, da dieser seitens des Trägers aufgenommenen Bindemittelmenge die Bindung der gesamten auf zunehmenden pulverförmigen Oxidmasse obliegt. Ein weiterer Nachteil der Methode besteht darin, daß der Befeuchtungsgrad der jeweiligen Oberflächenschicht während des Beschichtungsvorgangs ständig variiert. D.h. die Grundschicht trifft auf die Feuchte des unbeschichteten Trägers. Anschließend muß die Feuchtigkeit erst durch die Grundschicht an deren Oberfläche wandern, um weitere Aktivmasse anhaften zu können u.s.w.. Als Folge wird ein zwiebelartiger Schalenaufbau erhalten, wobei insbesondere die Aneinanderhaftung aufeinander folgender Schichten nicht zu befriedigen vermag. Einwirkung von Druck bedingt daher in der Regel ein aufeinanderfolgendes Abblättern der einzelnen Schichten. In allen Ausführungsbeispielen wird Wasser als alleiniges Bindemittel verwendet.

Die DE-A 29 09 671 versucht die Nachteile der ebenda beschriebenen verfahrensweise dadurch zu mindern, daß die kugelförmigen Trägerkörper in einen geneigten rotierenden Drehteller gefüllt werden. Der rotierende Drehteller führt die kugelförmigen Trägerkörper periodisch unter zwei in bestimmtem Abstand aufeinanderfolgend angeordneten Dosiervorrichtungen hindurch. Die erste der beiden Dosiervorrichtungen entspricht einer Düse, durch die die Trägerkugeln mit Wasser besprüht und kontrolliert befeuchtet werden. Die zweite Dosiervorrichtung befindet sich außerhalb des Zerstäubungskegels des eingesprühten Wassers und dient dazu, feinteilige oxidische Aktivmasse zuzuführen (z.B. über eine Schüttelrinne). Die kontrolliert befeuchteten Trägerkugeln nehmen das zugeführte Katalysatorpulver auf, das sich durch die rollende Bewegung auf der äußeren Oberfläche der Trägerkugeln zu einer zusammenhängenden Schale verdichtet. Die so grundbeschichtete Trägerkugel durchläuft als sozusagen neuer Trägerkörper im Verlauf der darauffolgenden Umdrehung wiederum die Sprühdüse, wird dabei in gleicher Weise kontrolliert befeuchtet, um im Verlauf der Weiterbewegung eine weitere Schicht feinteiliger oxidischer Aktivmasse aufnehmen zu können u.s.w.. Auf die beschriebene Art und Weise läßt sich die Schalendicke im wesentlichen gezielt einstellen. Auch wird die Homogenität des Schalenaufbaus verbessert. Durch Einleiten von Heißluft kann das als Bindemittel verwendete Wasser abschließend entfernt werden. Ein weiterer vorteil der beschriebenen Verfahrensweise ist, daß die zugeführte feinteilige oxidische Aktivmasse so bemessen werden kann, daß sie während der Beschichtung vollständig aufgenommen wird, so daß keine Aktivmassenverluste einhergehen. Nachteilig an der beschriebenen Verfahrensweise ist jedoch, daß die alleinige Verwendung des Bindemittels Wasser keine in vollem Umfang befriedigende Haftfestigkeit der Schale auf der Oberfläche der Trägerkugel zu bewirken vermag. Auch vermag in der Regel die spezifische aktive Oberfläche der resultierenden oxidischen Aktivmassenschale nicht voll zu befriedigen.

Aufgabe der vorliegenden Erfindung war daher ein Verfahren zur Herstellung eines Katalysators, bestehend aus einem Trägerkörper und einer auf der Oberfläche des Trägerkörpers aufgebrachten katalytisch aktiven Oxidmasse, das die Nachteile der Verfahren des Standes der Technik nicht aufweist und sich insbesondere zur Herstellung von ringförmigen oxidischen Schalenkatalysatoren eignet. Letztere sind im vergleich mit kugelförmigen Schalenkatalysatoren bei identischer Aktivmasse, Schalendicke und volumenspezifischer Aktivität insbesondere insofern von Vorteil, als sie einen geringeren Druckabfall längs der Festbettschüttung bewirken. D.h. bei gegebener Reaktorbelastung (die dem Reaktor pro Zeiteinheit zugeführte Menge Reaktionsgemisch) bauen sich geringere Partialdrücke der gasförmigen Reaktanden auf. Dies hat zur Folge, daß das bei exothermen katalytischen Festbett-Gasphasenoxidationen längs eines einzelnen Kontaktrohres in Strömungsrichtung üblicherweise durchlaufene Temperaturmaximum ("hot spot") eine reduzierte Amplitude aufweist. Dies wirkt sich vorteilhaft auf die Lebensdauer (Standzeit) der eingesetzten oxidischen Aktivmasse aus. Ein weiterer vorteil von ringförmigen Schalenkatalysatoren gegenüber solchen mit Kugelform ist, daß das Temperaturverhalten des Rohrbündelreaktors auf Schwankungen der Eintrittstemperatur des die Kontaktrohre umgebenden Temperiermediums weniger empfindlich reagiert. Erhöht sich diese Eintrittstemperatur beispielsweise zufällig um ein Grad, so nehmen die hot spot-Temperaturen in den Kontaktrohren üblicherweise um mehr als ein Grad zu. Dieser Mehranstieg ist bei ringförmigen Schalenkatalysatoren (identische Aktivmassen, Schalendicken und volumenspezifische Aktivität vorausgesetzt) jedoch weniger ausgeprägt.

Als Lösung der dieser Erfindung zugrunde liegenden Aufgabe wurde ein Verfahren zur Herstellung eines Katalysators, der aus einem Trägerkörper und einer auf der Oberfläche des Trägerkörpers aufgebrachten katalytisch aktiven Oxidmasse besteht, bei dem man den Trägerkörper zunächst mit einem flüssigen Bindemittel befeuchtet, danach durch Inkontaktbringen mit trockener, feinteiliger, aktiver Oxidmasse an der Oberfläche des befeuchteten Trägerkörpers eine Schicht aktiver Oxidmasse anheftet und anschließend das flüssige Bindemittel aus dem mit aktiver Oxidmasse beschichteten, befeuchteten Trägerkörper entfernt, gefunden, das dadurch gekennzeichnet ist, daß als flüssiges Bindemittel eine Lösung bestehend aus 20 bis 90 Gew.-% Wasser und 10 bis 80 Gew.-% einer organischen Verbindung, deren Siedepunkt oder Sublimationstemperatur bei Normaldruck (1 atm) > 100°C, vorzugsweise > 150°C beträgt, verwendet wird. Von der eben genannten Verfahrensweise wird in der US-A 4 034 060 zufällig zur Herstellung von Katalysatoren Gebrauch gemacht, bei denen zur Herstellung

a) als Träger Aluminiumoxid Pellets mit einem Durchmesser von 2 bis 4 mm,

b) als flüssiges Bindemittel eine 80 gew.-%ige wäßrige Milchsäurelösung und

c) als aktive Oxidmasse ein mit Barium dotiertes Cu-Cr-Al-Oxid

verwendet werden, weshalb diese Herstellweise der US-A 4 034 060 von dem in dieser Schrift Beanspruchten ausgenommen ist.

Vorzugsweise beträgt der organische Anteil an dem erfindungsgemäß zu verwendenden flüssigen Bindemittel 10 bis 50 und besonders bevorzugt 20 bis 30 Gew.-%.

Als organische Komponente des erfindungsgemäßen flüssigen Bindemittels eignen sich insbesondere ein- und mehrwertige organische Alkohle wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin, ein- oder mehrwertige organische Amide wie Formamid oder Monosaccharide und Oligosaccharide wie Glucose, Fructose, Saccharose oder Lactose. Bevorzugt sind solche organischen Komponenten, deren Siedepunkt oder Sublimationstemperatur bei Normaldruck unterhalb der Calcinationstemperatur, die zur Erzeugung der katalytisch aktiven Oxidmasse angewendet wurde, liegt, oder sich im Beisein von Sauerstoff an der katalytisch aktiven Oxidmasse unterhalb dieser Calcinationstemperatur in gasförmige Bestandteile zersetzen. Üblicherweise beträgt die Calcinationstemperatur ≤ 500, häufig ≤ 400 und vielfach ≤ 300°C. Besonders vorteilhaft sind erfindungsgemäß solche flüssigen Bindemittel, deren Siedepunkt bei Normaldruck oberhalb 100, vorzugsweise oberhalb 150°C liegt.

Die Vorteilhaftigkeit des erfindungsgemäßen Verfahrens, im Unterschied zu einer Verwendung von reinem Wasser als Bindemittel, wird, ohne Anspruch auf Gültigkeit, u.a. darauf zurückgeführt, daß die erfindungsgemäßen flüssigen Bindemittel sowohl die feinteiligen oxidischen Aktivmassen als auch die Trägerkörper besser zu benetzen vermögen.

Die Materialien der Trägerkörper sind vorzugsweise chemisch inert, d.h. sie greifen in den Ablauf der Gasphasenoxidation, die durch die erfindungsgemäß hergestellten Schalenkatalysatoren katalysiert wird, im wesentlichen nicht ein. Als Materialien für die Trägerkörper kommen erfindungsgemäß insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.

Mit Vorteil ist die Oberfläche des Trägerkörpers rauh, da eine erhöhte Oberflächenrauhigkeit in der Regel eine erhöhte Haftfestigkeit der aufgebrachten Schale an oxidischer Aktivmasse bedingt. Vorzugsweise liegt die Oberflächenrauhigkeit $R_z$ des Trägerkörpers im Bereich von 40 bis 200 μm, vorzugsweise 40 bis 100 μm (bestimmt gemäß DIN 4768 Blatt 1 mit einem "Hommel Tester für DIN-ISO Oberflächenmeßgrößen" der Fa. Hommelwerke). Die Trägermaterialien können porös oder unporös sein. Vorzugsweise ist das Trägermaterial unporös (Gesamtvolumen der Poren auf das Volumen des Trägerkörpers bezogen ≤ 1 Vol.-%).

Prinzipiell kommen für das erfindungsgemäße Verfahren beliebige Geometrien der Trägerkörper in Betracht. Ihre Längstausdehnung beträgt in der Regel 1 bis 10 mm. Vorzugsweise werden jedoch Kugeln oder Zylinder, insbesondere Hohlzylinder, als Trägerkörper angewendet.

Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Besonders bevorzugte ringförmige Trägerkörper besitzen eine Länge von 3 bis 6 mm, einen Außendurchmesser von 4 bis 8 mm und eine Wanddicke von 1 bis 2 mm. Ganz besonders bevorzugt sind Ringe der Geometrie 7 mm x 3 mm x 4 mm (Außendurchmesser x Länge x Innendurchmesser).

Die Dicke der erfindungsgemäß auf den Trägerkörper aufgebrachten katalytisch aktiven Oxidmasse liegt zweckmäßigerweise in der Regel bei 10 bis 1000 μm. Bevorzugt sind, insbesondere bei ringförmigen Trägerkörpern, 10 bis 500 μm, besonders bevorzugt 100 bis 500 μm und ganz besonders bevorzugt 200 bis 300 μm.

Die Feinheit der auf die Oberfläche des Trägerkörpers auf zubringenden katalytisch aktiven Oxidmasse wird selbstredend an die gewünschte Schalendicke angepaßt. Für den Vorzugsbereich einer Schalendicke von 100 bis 500 μm eignen sich insbesondere solche Aktivmassenpulver, von denen 50 % der Pulverpartikel ein Sieb der Maschenweite 1 bis 10 μm passieren und deren Anteil an Partikeln mit einer Längstausdehnung oberhalb von 50 μm weniger als 1 % beträgt. In der Regel entspricht die Verteilung der Längstausdehnungen der Pulverpartikel herstellungsbedingt einer Gaußverteilung.

Zur Erzielung der gewünschten Schalendicke wird das erfindungsgemäße verfahren in zweckmäßiger Weise periodisch wiederholt. D.h. der grundbeschichtete Trägerkörper bildet dann den in der darauffolgenden Periode in erfindungsgemäßer Weise zunächst zu befeuchtenden und dann durch Kontakt mit trockener feinteiliger oxidischer Aktivmasse zu beschichtenden "Trägerkörper" u.s.w..

Für eine Durchführung des erfindungsgemäßen Verfahrens im technischen Maßstab empfiehlt sich daher die Anwendung des in der DE-A 29 09 671 offenbarten Verfahrensprinzips, mit dem Unterschied, daß anstelle von Wasser ein erfindungsgemäßes flüssiges Bindemittel angewendet wird.

D.h. die zu beschichtenden Trägerkörper werden in einen vorzugsweise geneigten (der Neigungswinkel beträgt in der Regel 30 bis 90°) rotierenden Drehbehälter (z.B. Drehteller oder Dragierkessel) gefüllt. Der rotierende Drehbehälter führt die insbesondere kugelförmigen oder zylindrischen, vor allem hohlzylindrischen, Trägerkörper unter zwei in bestimmtem Abstand aufeinanderfolgend angeordneten Dosiervorrichtungen hindurch. Die erste der beiden Dosiervorrichtungen entspricht zweckmäßig einer Düse, durch die die im rotierenden Drehteller rollenden Trägerkörper mit dem erfindungsgemäß zu verwendenden flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse zuzuführen (z.B. über eine Schüttelrinne). Die kontrolliert befeuchteten Trägerkugeln nehmen das zugeführte Katalysatorpulver auf, das sich durch die rollende Bewegung auf der äußeren Oberfläche der zylinder- oder kugelförmigen Trägerkörper zu einer zusammenhängenden Schale verdichtet (im inneren Kreis eines hohlzylinderischen Trägerkörpers findet eine solche verdichtende Bewegung nicht statt, weshalb dieser im wesentlichen unbeschichtet bleibt).

Bei Bedarf durchläuft der so grundbeschichtete Trägerkörper im Verlauf der darauffolgenden Umdrehung wiederum die Sprühdüse, wird dabei kontrolliert befeuchtet, um im verlauf der Weiterbewegung eine weitere Schicht feinteiliger oxidischer Aktivmasse aufnehmen zu können u.s.w. (eine Zwischentrocknung ist in der Regel nicht erforderlich). Die Entfernung des erfindungsgemäß verwendeten flüssigen Bindemittels kann z.B. durch abschließende Wärmezufuhr, z.B. durch Einwirkung von heißen Gasen, wie $N_2$ oder Luft, erfolgen. Ein besonderer Vorzug der vorstehend beschriebenen Ausführungsform des erfindungsgemäßen Verfahrens besteht darin, daß in einem Arbeitsgang Schalenkatalysatoren mit schichtförmig aus zwei oder mehr unterschiedlichen aktiven oxidischen Massen bestehenden Schalen hergestellt werden können. Bemerkenswerterweise bewirkt das erfindungsgemäße Verfahren dabei sowohl eine voll befriedigende Haftung der aufeinanderfolgenden Schichten aneinander, als auch der Grundschicht auf der Oberfläche des Trägerkörpers. Dies gilt auch im Fall von ringförmigen Trägerkörpern.

Wesentlich für die vorbeschriebene Ausführungsform des erfindungsgemäßen Verfahrens ist, daß die Befeuchtung der zu beschichtenden Oberfläche des Trägerkörpers in kontrollierter Weise vorgenommen wird. Kurz ausgedrückt heißt dies, daß man die Trägeroberfläche zweckmäßig so befeuchtet, daß diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 29 09 671.

Ein Vorzug des erfindungsgemäßen Verfahrens besteht darin, daß die abschließende Entfernung des verwendeten flüssigen Bindemittels in kontrollierter Weise z.B. durch verdampfen und/oder Sublimieren vorgenommen werden kann. Im einfachsten Fall kann dies durch Einwirkung heißer Gase entsprechender Temperatur (häufig 50 bis 150°C) erfolgen. Durch Einwirkung heißer Gase kann aber auch nur eine Vortrocknung bewirkt werden. Die Endtrocknung kann dann beispielsweise in einem Trockenofen beliebiger Art (z.B. Bandtrockner) erfolgen. Die einwirkende Temperatur sollte nicht oberhalb der zur Herstellung der oxidischen Aktivmasse angewendeten Calcinationstemperatur liegen.

In überraschender Weise wurde gefunden, daß sich zahlreiche der polaren organischen Bindemittelkomponenten an oxidischen Massen bei erhöhten Temperaturen (die unterhalb der vorerwähnten Calcinationstemperatur liegen) und im Beisein von Luftsauerstoff zu gasförmigen Bestandteilen wie Ameisensäure, $H_2O$, $CO_2$ oder CO zersetzen. In überraschender Weise geht damit in der Regel keine Minderung der Haftfestigkeit der oxidischen Schale auf der Oberfläche des Trägerkörpers einher. Andererseits wird dabei jedoch eine Erhöhung der spezifischen Oberfläche der oxidischen Aktivmasse erzielt. Dadurch ist es für verschiedene oxidische Aktivmassen erstmals möglich, ringförmige Schalenkatalysatoren zur Verfügung zu stellen, die sowohl hinsichtlich der Haftfestigkeit der oxidischen Schale als auch hinsichtlich der spezifischen katalytisch aktiven Oberfläche der in der Schale befindlichen oxidischen Aktivmasse in vollem Umfang zu befriedigen vermögen.

Angaben hinsichtlich der spezifischen Oberfläche O ($m^2$/g) beziehen sich in dieser Schrift auf Ermittlungen nach DIN 66131 (Bestimmung der spezifischen Oberfläche von Feststoffen durch Gasadsorption ($N_2$) nach Brunauer-Emmet-Teller (BET)). Üblicherweise werden sie so durchgeführt, daß zunächst die Oberfläche für den unbeschichteten Trägerkörper und anschließend für den Schalenkatalysator ermittelt wird. Differenzbildung ergibt dann den gewünschten Wert für die oxidische Aktivmasse der Schale. Vielfach ist die oxidische Aktivmasse der Schale durch feinteiliges, katalytisch inertes Oxid (je nach Gasphasenreaktion häufig Oxide des Si, Al, Zr und/oder Ti) verdünnt. Wird in dieser Schrift von spezifischer Oberfläche der oxidischen Aktivmasse gesprochen, so bedeutet dies den Wert für O ausschließlich des Beitrags dieser inerten Verdünner. Da diese inerten Verdünner in der Regel bereits als solche bei der Katalysatorherstellung zugesetzt werden, läßt sich sowohl ihr Beitrag zur Oberfläche als auch zur Masse vorab ermitteln. O ergibt sich darin gemäß folgender Beziehung:

$$O = \frac{\text{Gesamtoberfläche der Schale - Oberfälche der in der Schale enthaltenen interten Verdünner}}{\text{Gesamtmasse Schale - Masse der in der Schale enthaltenen interten Verdünner}}$$

D.h. O meint die spezifische katalytisch aktive Oberfläche.

Durch geeignete Wahl des erfindungsgemäß zu verwendenden flüssigen Bindemittels lassen sich unabhängig von der Art der katalytisch aktiven oxidischen Masse nach dem erfindungsgemäßen Verfahren kugel- und ringförmige Schalenkatalysatoren (im beschriebenen Schalendickenbereich) erzeugen, deren Wert für O regelmäßig 20 bis 30 $m^2$/g beträgt. Diese Werte sind für ringförmige oxidische Schalenkatalysatoren ungewöhnlich hoch und nach Herstellverfahren gemäß des Standes der Technik höchstens für einen erhöhten Phosphoranteil aufweisende oxidische Aktivmassen vom Typ der Heteropolysäuren (Keggin-Struktur-Typ) bekannt.

Ein Maß für die Haftfestigkeit der oxidischen Schale ist der nachfolgende Drehteller-Abriebtest, auf den sich alle Haftfestigkeitsangaben in dieser Schrift beziehen.

Bei Raumtemperatur wird ein geneigter Drehteller (Neigungswinkel 45°) aus poliertem V2A Stahl (300 mm Durchmesser, 100 mm Randhöhe, keine Einbauten) zu 30 % seines Volumens mit den Schalenkatalysatoren gefüllt und 5 min mit einer Drehgeschwindigkeit von 35 Umdrehungen pro Minute gedreht. Der dabei erzeugte Abrieb an Aktivmasse wird gewogen. Dividiert durch die auf den eingefüllten Schalenkatalysatoren insgesamt befindliche oxidische Aktivmassenmenge und multipliziert mit 100 wird der Abrieb A in % erhalten.

Unabhängig von der Art der katalytisch aktiven oxidischen Masse werden nach dem erfindungsgemäßen Verfahren in der Regel, auch bei den vorerwähnten erhöhten Werten für O, kugel- oder ringförmige Schalenkatalysatoren erhalten, deren Wert für A < 10 Gew.-%, meist < 5 Gew.-% und bei Verwendung der in dieser Schrift als besonders bevorzugt genannten flüssigen Bindemittel sogar < 0,5 Gew.-% beträgt.

Es sei an dieser Stelle erwähnt, daß alle Verfahrensschritte im Rahmen der erfindungsgemäßen Beschichtung der Trägerkörper mit oxidischer Aktivmasse, vorab der Entfernung des flüssigen Bindemittels, in der Regel bei Raumtemperatur (d.h. ca. 25$^0$C) erfolgen.

Wesentliches Merkmal der erfindungsgemäßen Verfahrens ist, daß auf den Trägerkörper keine Vorläufermasse sondern die katalytisch aktive oxidische Masse als solche aufgetragen wird. Zur Herstellung derselben geht man üblicherweise von in an sich bekannter Weise geeigneten Quellen der katalytisch aktiven, oxidischen Masse aus und erzeugt aus diesen ein möglichst inniges, vorzugsweise feinteiliges, Trockengemisch, welches dann der Calcinierung unterworfen und gegebenenfalls durch Mahlen in feinteilige Form überführt wird. Wesentlich ist, wie allgemein bekannt, nur, daß es sich bei den Quellen entweder bereits um Oxide handelt, oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht.

Das innige Vermischen der Ausgangsverbindungen kann in trockener oder in nasser Form erfolgen. Erfolgt es in trockener Form, werden die Ausgangsverbindungen zweckmäßigerweise als feinteilige Pulver eingesetzt und nach Mischen und gegebenenfalls Verpressen der Calcinierung unterworfen. Vorzugsweise erfolgt das innige Vermischen jedoch in nasser Form. Üblicherweise werden die Ausgangsverbindungen dabei in Form einer wäßrigen Lösung oder Suspension miteinander vermischt. Anschließend wird die wäßrige Masse getrocknet und nach Trocknung calciniert. Vorzugsweise erfolgt der Trocknungsprozeß durch Sprühtrocknung. Das dabei anfallende Pulver erweist sich für eine unmittelbare Weiterverarbeitung häufig als zu feinteilig. In diesen Fällen kann es unter Zusatz von Wasser geknetet werden. Die anfallende Knetmasse wird anschließend der Calcinierung unterworfen und danach zu einer feinteiligen oxidischen Aktivmasse gemahlen.

Die Calcinationsbedingungen sind dem Fachmann für die verschiedenen möglichen oxidischen Aktivmassen an sich bekannt.

Günstig ist das erfindungsgemäße Verfahren im Fall von Mo und V bzw. Mo, Fe und Bi enthaltenden Multimetalloxidmassen.

Als besonders vorteilhaft erweist sich das erfindungsgemäße Verfahren im Fall von als Schale aufzubringenden aktiven Multimetalloxiden der allgemeinen Stöchiometrie I

$$Mo_{12}V_aX^1_bX^2_cX^3_dX^4_eX^5_fX^6_gO_n \tag{I},$$

in der die Variablen folgende Bedeutung haben:

$X^1$    W, Nb, Ta, Cr und/oder Ce,
$X^2$    Cu, Ni, Co, Fe, Mn und/oder Zn,
$X^3$    Sb und/oder Bi,
$X^4$    wenigstens eines oder mehrere Alkalimetalle,
$X^5$    wenigstens eines oder mehrere Erdalkalimetalle,
$X^6$    Si, Al, Ti und/oder Zr,
a    1 bis 6,
b    0,2 bis 4,
c    0,5 bis 18,
d    0 bis 40,
e    0 bis 2,
f    0 bis 4,
g    0 bis 40 und
n    eine Zahl, die durch die Wertigkeit und Häufigkeit der von Sauerstoff verschiedenen Elemente in I bestimmt wird.

Die Herstellung von aktiven Multimetalloxiden I einschließlich der Calcinationsbedingungen beschreibt die DE-A 43 35 973. Die DE-A 43 35 973 offenbart auch bevorzugte Ausführungsformen innerhalb der aktiven Multimetalloxide I. Zu diesen zählen beispielsweise jene Multimetalloxide I, die von nachfolgenden Bedeutungen der Variablen der allgemeinen Formel I erfaßt werden:

| | |
|---|---|
| $X^1$ | W, Nb und/oder Cr, |
| $X^2$ | Cu, Ni, Co und/oder Fe, |
| $X^3$ | Sb, |
| $X^4$ | Na und/oder K, |
| $X^5$ | Ca, Sr und/oder Ba, |
| $X^6$ | Si, Al und/oder Ti, |
| a | 2,5 bis 5, |
| b | 0,5 bis 2, |
| c | 0,5 bis 3, |
| d | 0 bis 2, |
| e | 0 bis 0,2, |
| f | 0 bis 1, |
| g | 0 bis 15 und |
| n | eine Zahl, die durch die Wertigkeit und Häufigkeit der von Sauerstoff verschiedenen Elemente in I bestimmt wird. |

Ganz besonders bevorzugte Multimetalloxide I sind jedoch jene der allgemeinen Formel I'

$$Mo_{12}V_a X_b^1 X_c^2 X_f^5 X_g^6 O_n \tag{I'},$$

mit

| | |
|---|---|
| $X^1$ | W und/oder Nb, |
| $X^2$ | Cu und/oder Ni, |
| $X^5$ | Ca und/oder Sr, |
| $X^6$ | Si und/oder Al, |
| a | 3 bis 4,5, |
| b | 1 bis 1,5, |
| c | 0,75 bis 2,5, |
| f | 0 bis 0,5, |
| g | 0 bis 8 und |
| n | eine Zahl, die durch die Wertigkeit und Häufigkeit der von Sauerstoff verschiedenen Elemente in I' bestimmt wird. |

Mit den aktiven Multimetalloxiden I erfindungsgemäß hergestellte Schalenkatalysatoren eignen sich insbesondere zur gasphasenkatalytisch oxidativen Herstellung von Acrylsäure aus Acrolein. Dies gilt insbesondere für kugel- oder ringförmige Schalenkatalysatoren. Vor allem dann, wenn diese die in dieser Schrift als bevorzugt beschriebene Charakteristik (Geometrie, Schalendicke etc.) aufweisen. Die allgemeinen Reaktionsbedingungen für die gasphasenkatalytische Oxidation von Acrolein zu Acrylsäure finden sich ebenfalls in der DE-A 43 35 973.

Das erfindungsgemäße Verfahren eignet sich auch im Fall von aktiven Multimetalloxiden wie sie für die katalytische Gasphasenoxidation von Methacrolein zu Methacrylsäure angewendet werden und z.B. in der DE-A 40 22 212 beschrieben sind.

Weiterhin erweist sich das erfindungsgemäße Verfahren im Fall von als Schale aufzubringenden aktiven Multimetalloxiden der allgemeinen Stöchiometrie II

$$Mo_{12}Bi_a Fe_b X_c^1 X_d^2 X_e^3 X_f^4 O_n \tag{II},$$

in der die Variablen nachfolgende Bedeutung aufweisen:

| | |
|---|---|
| $X^1$ | Nickel und/oder Kobalt, |
| $X^2$ | Thallium, ein Alkalimetall und/oder ein Erdalkalimetall, |
| $X^3$ | Phosphor, Arsen, Bor, Antimon, Zinn, Cer, Blei, Niob und/oder Wolfram, |
| $X^4$ | Silicium, Aluminium, Titan und/oder Zirkonium, |
| a | 0,5 bis 5, |
| b | 0,01 bis 3, |

c      3 bis 10,

d      0,02 bis 2,

e      0 bis 5,

f      0 bis 10 und

n      eine Zahl, die durch die Wertigkeit und Häufigkeit der von Sauerstoff verschiedenen Elemente in II bestimmt wird, als geeignet.

Die Herstellung von aktiven Multimetalloxiden II einschließlich der Calcinationsbedingungen beschreibt die DE-A 40 23 239.

Mit den aktiven Multimetalloxiden II erfindungsgemäß hergestellte Schalenkatalysatoren eignen sich insbesondere zur gasphasenkatalytisch oxidativen Herstellung von Acrolein aus Propen. Dies gilt insbesondere für kugel- oder ringförmige Schalenkatalysatoren. Vor allem dann, wenn diese die in dieser Schrift als bevorzugt beschriebene Charakteristik (Geometrie, Schalendicke etc.) aufweisen. Die allgemeinen Reaktionsbedingungen für die gasphasenkatalytische Oxidation von Propen zu Acrolein finden sich ebenfalls in der DE-A 40 23 239 sowie in der DE-A 44 31 957.

Die vorgenannten Schalenkatalysatoren der aktiven Multimetalloxide II eignen sich aber auch zur gasphasenkatalytisch oxidativen Herstellung von Methacrolein aus tert.-Butanol, iso-Butan, iso-Buten oder tert.-Butylmethylether. Die allgemeinen Reaktionsbedingungen für diese katalytische Gasphasenoxidation finden sich z.B. in der DE-A 40 23 239 und in der DE-A 43 35 172.

Weiterhin eignet sich das erfindungsgemäße Verfahren im Fall der aktiven Oxidmassen der DE-A 44 05 514.

Selbstverständlich eignet sich das erfindungsgemäße Verfahren aber ganz generell zur Herstellung von Schalenkatalysatoren auf der Basis aktiver Oxidmassen, insbesondere für die in dieser Schrift im Rahmen der Würdigung des Standes der Technik aufgeführten katalytischen Gasphasenoxidationen. Dies gilt auch dann, wenn die oxidische Aktivmasse neben Sauerstoff lediglich ein anderes Element umfaßt.

Die Vorteile des erfindungsgemäßen Verfahrens liegen insbesondere in

-      der variabel einstellbaren Schalendicke,

-      einer hohen Haftfestigkeit der oxidischen Aktivmasse bei gleichzeitig voll befriedigender spezifischer Oberfläche derselben,

-      einer erhöhten Homogenität der resultierenden Schalendicke sowohl über die Oberfläche eines als auch verschiedener Trägerkörper betrachtet und

-      einer befriedigenden Produktionsleistung des Herstellverfahrens.

Dies gilt insbesondere im Fall von ringförmigen Trägerkörpern.

Beispiele

a) Herstellung von katalytisch aktiven Oxidmassen

A: Katalytisch aktive Oxidmasse $Mo_{12}V_3W_{1,2}Cu_{2,4}O_n$

190 g Kupfer(II)acetatmonohydrat wurden in 2700 g Wasser zu einer Lösung I gelöst. In 5500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und das wäßrige Gemisch bei einer Austrittstemperatur von 110°C sprühgetrocknet. Danach wurde das Sprühpulver je kg Pulver mit 0,15 kg Wasser verknetet.

Der so erhaltene Katalysatorvorläufer wurde in einem mit einem Sauerstoff/Stickstoff-Gemisch beschickten Umluftofen calciniert. Der Sauerstoffgehalt wurde dabei so eingestellt, daß am Ausgang des Umluftofens ein $O_2$-Gehalt von 1,5 Vol.-% bestand. Im Rahmen der Calcinierung wurde die Knetmasse zunächst mit einer Geschwindigkeit von 10 K/min auf 300°C aufgeheizt und anschließend während 6 h auf dieser Temperatur gehalten. Danach wurde mit einer Geschwindigkeit von 10 K/min auf 400°C aufgeheizt und diese Temperatur noch 1 h aufrechterhalten. Zur Realisierung eines bestimmten Ammoniakgehalts der Calcinierungsatmosphäre wurden die Ofenbeladung B (g Katalysatorvorläufer pro l Innenvolumen des Umluftofens), der Eingangsvolumenstrom ES (Nl/h) des Sauerstoff/Stickstoff -Gemisches und die Verweilzeit VZ (sec) der Sauerstoff/Stickstoff-Beschickung (Verhältnis aus Innenvolumen des Umluftofens und Volumenstrom des zugeführten Sauerstoff/Stickstoff-Gemisches) wie folgt gewählt:

| B: | 250 g/l; |
|---|---|
| ES: | 80 Nl/h; |
| VZ: | 135 sec. |

Der verwendete Umluftofen wies ein Innenvolumen von 3 l auf. Das calcinierte katalytisch aktive Material wurde zu einem feinteiligen Pulver gemahlen, von dem 50 % der Pulverpartikel ein Sieb der Maschenweite 1 bis 10 $\mu$m passierten und dessen Anteil an Partikel mit einer Längstausdehnung oberhalb von 50 $\mu$m weniger als 1 % betrug.

B: Katalytisch aktive Oxidmasse $Mo_{12}V_3W_{1,2}Cu_{1,6}Ni_{0,8}O_n$

128 g Kupfer(II)acetatmonohydrat und 81 g Nickel(II)acetattetrahydrat wurden in 2700 g Wasser zu einer Lösung I gelöst. In 5500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und das wäßrige Gemisch bei einer Austrittstemperatur von 110°C sprühgetrocknet. Danach wurde das Sprühpulver je kg Pulver mit 0,15 kg Wasser verknetet. Die Knetmasse wurde in einem mit Luft beschickten Drehrohrofen innerhalb von 3 h auf 400°C aufgeheizt und anschließend 5 h bei 400°C calciniert. Danach wurde das calcinierte, katalytisch aktive Material zu einem feinteiligen Pulver gemahlen, von dem 50 % der Pulverpartikel ein Sieb der Maschenweite 1 bis 10 $\mu$m passierten und dessen Anteil an Partikeln mit einer Längsausdehnung oberhalb von 50 $\mu$m weniger als 1 % betrug.

b) Herstellung von Schalenkatalysatoren

VS1: 28 kg ringförmiger Trägerkörper (7 mm Außendurchmesser, 3 mm Länge, 4 mm Innendurchmesser, Steatit, mit einer Oberflächenrauhigkeit $R_z$ von 45 $\mu$m und mit einem auf das Volumen der Trägerkörper bezogenen Porengesamtvolumen $\leq$ 1 Vol.-%, Hersteller: Hoechst Ceramtec, DE) wurden in einem Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse wurden innerhalb von 25 min 2000 g Wasser auf die Trägerkörper aufgesprüht. Gleichzeitig wurden im selben Zeitraum 10,35 kg des katalytisch aktiven Oxidpulvers aA) über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und Wasser wurde bei einer Drehgeschwindigkeit von 2 U/min 20 min 110°C heiße Luft in den Dragierkessel geblasen. Anschließend wurde noch 2 h bei 250°C in ruhender Schüttung (Hordenofen) unter Luft getrocknet. Es wurden ringförmige Schalenkatalysatoren erhalten, deren Anteil an oxidischer Aktivmasse, bezogen auf die Gesamtmasse, 27 Gew.-% betrug. Die Schalendicke lag, sowohl über die Oberfläche eines Trägerkörpers als auch über die Oberfläche verschiedener Trägerkörper betrachtet, bei 230 $\pm$ 50 $\mu$m.

S1: Wie VS1, anstelle von 2000 g Wasser wurden jedoch 2000 g einer aus 75 Gew.-% $H_2O$ und 25 Gew.-% Glycerin bestehenden wäßrigen Lösung eingesetzt. Die resultierenden ringförmigen Schalenkatalysatoren wiesen denselben oxidischen Aktivmassenanteil wie bei SV1 auf, die Schwankungsbreite der Schalendicke lag jedoch bei 230 $\pm$ 25 $\mu$m.

S2: Wie VS1, anstelle von 2000 g Wasser wurden jedoch 2000 g einer aus 75 Gew.-% $H_2O$ und 25 Gew.-% Propionsäure bestehenden wäßrigen Lösung eingesetzt. Die resultierenden ringförmigen Schalenkatalysatoren wiesen denselben oxidischen Aktivmassenanteil wie bei SV1 auf, die Schwankungsbreite der Schalendicke lag jedoch bei 230 $\pm$ 30 $\mu$m.

S3: Wie VS1, anstelle von 2000 g Wasser wurden jedoch 2000 g einer aus 75 Gew.-% $H_2O$ und 25 Gew.-% Formamid bestehenden wäßrigen Lösung eingesetzt. Die resultierenden ringförmigen Schalenkatalysatoren wiesen denselben oxidischen Aktivmassenanteil wie bei SV1 auf, die Schwankungsbreite der Schalendicke lag jedoch bei 230 $\pm$ 30 $\mu$m.

S4: Wie VS1, anstelle von 2000 g Wasser wurden jedoch 2000 g einer aus 75 Gew.-% $H_2O$ und 25 Gew.-% Ethylenglykol bestehenden wäßrigen Lösung eingesetzt. Die resultierenden ringförmigen Schalenkatalysatoren wiesen denselben oxidischen Aktivmassenanteil wie bei SV1 auf, die Schwankungsbreite der Schalendicke lag jedoch bei 230 $\pm$ 25 $\mu$m.

S5: Wie VS1, anstelle von 2000 g Wasser wurden jedoch 2000 g einer aus 75 Gew.-% $H_2O$ und 25 Gew.-% 1,4-Butandiol bestehenden wäßrigen Lösung eingesetzt. Die resultierenden ringförmigen Schalenkatalysatoren wiesen denselben oxidischen Aktivmassenanteil wie bei SV1 auf, die Schwankungsbreite der Schalendicke lag jedoch bei 230 ± 30 μm.

S6: Wie VS1, anstelle von 2000 g Wasser wurden jedoch 2000 g einer aus 75 Gew.-% $H_2O$ und 25 Gew.-% 1,6-Hexandiol bestehenden wäßrigen Lösung eingesetzt. Die resultierenden ringförmigen Schalenkatalysatoren wiesen denselben oxidischen Aktivmassenanteil wie bei SV1 auf, die Schwankungsbreite der Schalendicke lag jedoch bei 230 ± 25 μm.

S7: Wie S1, anstelle von 10,35 kg des katalytisch aktiven Oxidpulvers aA) wurde jedoch die entsprechende Menge des katalytisch aktiven Oxidpulvers aB) eingesetzt. Die resultierenden ringförmigen Schalenkatalysatoren wiesen denselben oxidischen Aktivmassenanteil wie bei S1 auf, die Schwankungsbreite der Schalendicke lag ebenfalls bei 230 ± 25 μm.

VS2: Wie S1, anstelle von 2000 g Wasser wurden jedoch 2000 g Ethylenglykol eingesetzt. Es wurden keine getrennten, mit Aktivmasse beschichteten, Trägerkörper erhalten. Vielmehr erfolgte die Bildung stark aneinanderhaftender Mehrlingskörper.

VS3: Beispiel 1b) der DE-A 29 09 670 wurde nachgearbeitet.

c) Bestimmung der spezifischen, katalytisch aktiven Oberfläche O ($m^2$/g) sowie des Abriebs A (%) für Schalenkatalysatoren aus b)

Nachfolgende Tabelle 1 enthält die ermittelten Werte für O und A.

Tabelle 1

| Schalenkatalysator | O ($m^2$/g) | A (%) |
|---|---|---|
| VS1 | 17,5 | > 10 |
| S1 | 23,2 | 0,1 |
| S2 | 17,5 | 0,3 |
| S3 | 17,4 | 0,2 |
| S4 | 23,7 | 0,1 |
| S5 | 24,3 | 0,3 |
| S6 | 28,5 | 0,2 |
| S7 | 23,0 | 0,2 |
| VS3 | 17,4 | 0,1 |

d) Verfahren zur gasphasenkatalytisch oxidativen Herstellung von Acrylsäure aus Acrolein

Die Schalenkatalysatoren VS1 und S1 wurden wie folgt in einem von einem Salzbad umspülten Modellkontaktrohr getestet:

Modellkontaktrohr: V2A-Stahl, 2 mm Wandstärke, 25 mm Innendurchmesser; 1,5 l des Modellkontaktrohres wurden mit dem jeweiligen Schalenkatalysator gefüllt. Das Reaktionsgasgemisch wies folgende Ausgangszusammensetzung auf:

5 Vol.-% Acrolein,
7 Vol.-% Sauerstoff,
10 Vol.-% Wasserdampf,
78 Vol.-% Stickstoff.

Das Modellkontaktrohr wurde mit 3600 Nl/h an Reaktionsgasausgangsgemisch belastet. Die Temperatur des Salzbads wurde so eingestellt, daß bei einfachem Durchgang ein Acroleinumsatz von 99 mol-% resultierte.

Die diesbezüglich erforderliche Salzbadtemperatur T sowie die Selektivität S der Acrylsäurebildung zeigt die nachfolgende Tabelle 2

Tabelle 2

| Verwendeter Schalenkatalysator | T [°C] | S [mol-%] |
|---|---|---|
| VS1 | 267 | 95,2 |
| S1 | 263 | 95,3 |

**Patentansprüche**

1. Verfahren zur Herstellung eines Katalysators, der aus einem Trägerkörper und einer auf der Oberfläche des Trägerkörpers aufgebrachten katalytisch aktiven Oxidmasse besteht, bei dem man den Trägerkörper zunächst mit einem flüssigen Bindemittel befeuchtet, danach durch Inkontaktbringen mit trockener, feinteiliger, aktiver Oxidmasse an der Oberfläche des befeuchteten Trägerkörpers eine Schicht aktiver Oxidmasse anheftet und anschließend das flüssige Bindemittel aus dem mit aktiver Oxidmasse beschichteten befeuchteten Trägerkörper entfernt, dadurch gekennzeichnet, daß als flüssiges Bindemittel eine Lösung, bestehend aus 20 bis 90 Gew.-% Wasser und 10 bis 80 Gew.-% einer organischen Verbindung, deren Siedepunkt oder Sublimationstemperatur bei Normaldruck (1 atm) 100°C beträgt, verwendet wird, ausgenommen die Herstellung von Katalysatoren, bei denen zur Herstellung

    a) als Träger Aluminiumoxid Pellets mit einem Durchmesser von 2 bis 4 mm,

    b) als flüssiges Bindemittel eine 80 gew.-%ige wäßrige Milchsäurelösung und

    c) als aktive Oxidmasse ein mit Barium dotiertes Cu-Cr-Al-Oxid

    verwendet werden.

2. Verfahren nach Anspruch 1, dadurch gekennzeichnet, daß der Anteil der organischen Verbindung an dem flüssigen Bindemittel 10 bis 50 Gew.-% beträgt.

3. Verfahren nach Anspruch 1, dadurch gekennzeichnet, daß der Anteil der organischen Verbindung an dem flüssigen Bindemittel 20 bis 30 Gew.-% beträgt.

4. Verfahren nach Anspruch 1 bis 3, dadurch gekennzeichnet, daß die organische Verbindung wenigstens eine Verbindung aus der Gruppe umfassend ein- und mehrwertige Alkohole, ein- oder mehrwertige organische Carbonsäuren, Aminoalkohole, ein- oder mehrwertige organische Amide, Monosaccharide und Oligosaccharide ist.

5. Verfahren nach Anspruch 1 bis 4, dadurch gekennzeichnet, daß die organische Verbindung wenigstens eine Verbindung aus der Gruppe umfassend Ethylenglykol, 1,4-Butandiol, 1,6-Hexandiol, Glycerin, Propionsäure, Oxalsäure, Malonsäure, Glutarsäure, Maleinsäure, Ethanolamin, Diethanolamin, Formamid, Glucose, Fructose, Saccharose oder Lactose ist.

6. Verfahren nach Anspruch 1 bis 5, dadurch gekennzeichnet, daß der Siedepunkt des flüssigen Bindemittels bei Normaldruck (1 atm) > 150°C beträgt.

7. Verfahren nach Anspruch 1 bis 6, dadurch gekennzeichnet, daß der Trägerkörper aus Aluminiumoxid, Siliciumdioxid, Ton, Kaolin, Steatit, Bims, Aluminiumsilicat, Magnesiumsilicat, Siliciumcarbid, Zirkondioxid oder Thoriumdioxid besteht.

8. Verfahren nach Anspruch 1 bis 7, dadurch gekennzeichnet, daß die Oberflächenrauhigkeit $R_z$ des Trägerkörpers 40 bis 100 μm beträgt.

9. Verfahren nach Anspruch 1 bis 8, dadurch gekennzeichnet, daß das Gesamtvolumen der Poren des Trägerkörpers auf das Volumen des Trägerkörpers bezogen ≤ 1 Vol.-% beträgt.

10. Verfahren nach Anspruch 1 bis 9, dadurch gekennzeichnet, daß die Längstausdehnung des Trägerkörpers 1 bis 10 mm beträgt.

11. Verfahren nach Anspruch 1 bis 10, dadurch gekennzeichnet, daß als Trägerkörper Kugeln verwendet werden.

12. Verfahren nach Anspruch 1 bis 10, dadurch gekennzeichnet, daß als Trägerkörper Hohlzylinder verwendet werden.

13. Verfahren nach Anspruch 12, dadurch gekennzeichnet, daß die Hohlzylinder eine Länge von 2 bis 10 mm, einen Außendurchmesser von 4 bis 10 mm und eine Wanddicke von 1 bis 4 mm aufweisen.

14. Verfahren nach Anspruch 12, dadurch gekennzeichnet, daß die Hohlzylinder eine Länge von 3 bis 6 mm, einen Außendurchmesser von 4 bis 8 mm und eine Wanddicke von 1 bis 2 mm aufweisen.

15. Verfahren nach Anspruch 1 bis 14, dadurch gekennzeichnet, daß die Schichtdicke der auf die Oberfläche des Trägerkörpers aufgebrachten katalytisch aktiven Oxidmasse 10 bis 1000 $\mu$m beträgt.

16. Verfahren nach Anspruch 1 bis 14, dadurch gekennzeichnet, daß die Schichtdicke der auf die Oberfläche des Trägerkörpers aufgebrachten katalytisch aktiven Oxidmasse 100 bis 500 $\mu$m beträgt.

17. Verfahren zur Herstellung eines Katalysators, der aus einem Trägerkörper und einer auf der Oberfläche des Trägerkörpers aufgebrachten katalytisch aktiven Oxidmasse besteht, dadurch gekennzeichnet, daß das Verfahren gemäß Anspruch 1 bis 16 in periodischer Weise wiederholt angewendet wird, d.h. der grundbeschichtete Trägerkörper bildet den in der darauffolgenden Periode zu befeuchtenden und dann durch Kontakt mit trockener, feinteiliger, oxidischer Aktivmasse zu beschichtenden "Trägerkörper" u.s.w..

18. Verfahren nach Anspruch 17, dadurch gekennzeichnet, daß die zu beschichtenden Trägerkörper in einen rotierenden Drehbehälter gefüllt werden, der die Trägerkörper periodisch unter zwei aufeinanderfolgend angeordneten Dosiervorrichtungen hindurchführt, von denen die erste die im rotierenden Drehbehälter rollenden Trägerkörper mit dem flüssigen Bindemittel besprüht und die zweite die katalytisch aktive Oxidmasse in feinteiliger Form zudosiert.

19. Verfahren nach Anspruch 1 bis 18, dadurch gekennzeichnet, daß die katalytisch aktive Oxidmasse ein Mo und V enthaltendes Multimetalloxid ist.

20. Verfahren nach Anspruch 1 bis 18, dadurch gekennzeichnet, daß die katalytisch aktive Oxidmasse ein Mo, Fe und Bi enthaltendes Multimetalloxid ist.

21. Schalenkatalysator bestehend aus einem hohlzylindrischen Trägerkörper einer Länge von 2 bis 10 mm, einem Außendurchmesser von 4 bis 10 mm und einer Wanddicke von 1 bis 4 mm, sowie einer auf die äußere Oberfläche des Trägerkörpers aufgebrachten katalytisch aktiven Oxidmasse der allgemeinen Formel I

$$Mo_{12}V_a X_b^1 X_c^2 X_d^3 X_e^4 X_f^5 X_g^6 O_n \qquad (I),$$

in der die Variablen folgende Bedeutung haben:

| | |
|---|---|
| $X^1$ | W, Nb, Ta, Cr und/oder Ce, |
| $X^2$ | Cu, Ni, Co, Fe, Mn und/oder Zn, |
| $X^3$ | Sb und/oder Bi, |
| $X^4$ | wenigstens eines oder mehrere Alkalimetalle, |
| $X^5$ | wenigstens eines oder mehrere Erdalkalimetalle, |
| $X^6$ | Si, Al, Ti und/oder Zr, |
| a | 1 bis 6, |
| b | 0,2 bis 4, |
| c | 0,5 bis 18, |
| d | 0 bis 40, |
| e | 0 bis 2, |
| f | 0 bis 4, |
| g | 0 bis 40 und |

n      eine Zahl, die durch die Wertigkeit und Häufigkeit der von Sauerstoff verschiedenen Elemente in I bestimmt wird,

wobei die aufgebrachte katalytisch aktive Oxidmasse

- in einer Schichtdicke von 10 bis 1000 $\mu$m aufgebracht ist,

- eine spezifische, katalytisch aktive Oberfläche von 20 bis 30 m$^2$/g und

- im Drehteller-Abriebtest einen Abrieb < 10 Gew.-%, vorzugsweise < 5 Gew.-% und besonders bevorzugt < 0,5 Gew.-% aufweist.

22. Verfahren zur Herstellung von Acrylsäure durch gasphasenkatalytische Oxidation von Acrolein, dadurch gekennzeichnet, daß als Katalysator ein Schalenkatalysator gemäß Anspruch 21 mitverwendet wird.

23. Schalenkatalysator bestehend aus einem hohlzylindrischen Trägerkörper einer Länge von 2 bis 10 mm, einem Außendurchmesser von 4 bis 10 mm und einer Wanddicke von 1 bis 4 mm, sowie einer auf die äußere Oberfläche des Trägerkörpers aufgebrachten katalytisch aktiven Oxidmasse der allgemeinen Formel II

$$Mo_{12}Bi_aFe_bX^1_c\,X^2_d\,X^3_e\,X^4_f\,O_n \qquad\qquad\qquad (II),$$

in der die Variablen nachfolgende Bedeutung aufweisen:

$X^1$      Nickel und/oder Kobalt,
$X^2$      Thallium, ein Alkalimetall und/oder ein Erdalkalimetall,
$X^3$      Phosphor, Arsen, Bor, Antimon, Zinn, Cer, Blei, Niob und/oder Wolfram,
$X^4$      Silicium, Aluminium, Titan und/oder Zirkonium,
a      0,5 bis 5,
b      0,01 bis 3,
c      3 bis 10,
d      0,02 bis 2,
e      0 bis 5,
f      0 bis 10 und
n      eine Zahl, die durch die Wertigkeit und Häufigkeit der von Sauerstoff verschiedenen Elemente in II bestimmt wird,

wobei die aufgebrachte katalytisch aktive Oxidmasse

- in einer Schichtdicke von 10 bis 1000 $\mu$m aufgebracht ist,
- eine spezifische katalytische Oberfläche von 20 bis 30 m$^2$/g und
- im Drehteller-Abriebtest einen Abrieb < 10 Gew.-%, vorzugsweise < 5 Gew.-% und besonders bevorzugt < 0,5 Gew.-% aufweist.

24. Verfahren zur Herstellung von Acrolein oder Methacrolein durch gasphasenkatalytische Oxidation von Propen oder tert.-Butanol oder iso-Butan oder iso-Buten oder tert.-Butylmethylether, dadurch gekennzeichnet, daß als Katalysator ein Schalenkatalysator gemäß Anspruch 23 mitverwendet wird.

25. Verfahren der katalytischen Gasphasenoxidation, dadurch gekennzeichnet, daß als Katalysator das Produkt eines Verfahrens gemäß Anspruch 1 bis 20 verwendet wird.

26. Schalenkatalysator, bestehend aus einem Trägerkörper und einer auf der Oberfläche des Trägerkörpers aufgebrachten katalytisch aktiven Oxidmasse, erhältlich nach einem Verfahren gemäß Anspruch 1 bis 20.

**Claims**

1. A process for the preparation of a catalyst which consists of a carrier and a catalytically active oxide material applied to the surface of the carrier, in which the carrier is first moistened with a liquid binder, a layer of active oxide material is then bound to the surface of the moistened carrier by bringing it into contact with dry, finely divided, active oxide material, and the liquid binder is then removed from the moistened carrier coated with active oxide material, wherein the liquid binder used is a solution consisting of from 20 to 90% by weight of water and from 10 to 80% by weight of an organic compound whose boiling point or sublimation temperature at atmospheric pressure (1 atm) is > 100°C, excluding the preparation of catalysts in whose preparation

    a) aluminum oxide pellets having a diameter of from 2 to 4 mm are used as the carrier,

    b) an 80% strength by weight aqueous lactic acid solution is used as the liquid binder and

    c) a barium-doped Cu-Cr-Al oxide is used as the active oxide material.

2. A process as claimed in claim 1, wherein the liquid binder contains from 10 to 50% by weight of the organic compound.

3. A process as claimed in claim 1, wherein the liquid binder contains from 20 to 30% by weight of the organic compound.

4. A process as claimed in any of claims 1 to 3, wherein the organic compound is at least one compound selected from the group consisting of monohydric and polyhydric alcohols, monobasic and polybasic organic carboxylic acids, amino alcohols, monofunctional and polyfunctional organic amides, monosaccharides and oligosaccharides.

5. A process as claimed in any of claims 1 to 4, wherein the organic compound is at least one compound selected from the group consisting of ethylene glycol, 1,4-butanediol, 1,6-hexanediol, glycerol, propionic acid, oxalic acid, malonic acid, glutaric acid, maleic acid, ethanolamine, diethanolamine, formamide, glucose, fructose, sucrose and lactose.

6. A process as claimed in any of claims 1 to 5, wherein the boiling point of the liquid binder at atmospheric pressure (1 atm) is > 150°C.

7. A process as claimed in any of claims 1 to 6, wherein the carrier consists of alumina, silica, clay, kaolin, steatite, pumice, aluminum silicate, magnesium silicate, silicon carbide, zirconium dioxide or thorium dioxide.

8. A process as claimed in any of claims 1 to 7, wherein the surface roughness $R_z$ of the carrier is from 40 to 100 $\mu$m.

9. A process as claimed in any of claims 1 to 8, wherein the total volume of the pores of the carrier is $\leq$ 1% by volume, based on the volume of the carrier.

10. A process as claimed in any of claims 1 to 9, wherein the longest dimension of the carrier is from 1 to 10 mm.

11. A process as claimed in any of claims 1 to 10, wherein the carrier used comprises spheres.

12. A process as claimed in any of claims 1 to 10, wherein the carrier used comprises hollow cylinders.

13. A process as claimed in claim 12, wherein the hollow cylinders have a length of from 2 to 10 mm, an external diameter of from 4 to 10 mm and a wall thickness of from 1 to 4 mm.

14. A process as claimed in claim 12, wherein the hollow cylinders have a length of from 3 to 6 mm, an external diameter of from 4 to 8 mm and a wall thickness of from 1 to 2 mm.

15. A process as claimed in any of claims 1 to 14, wherein the coat thickness of the catalytically active oxide material applied to the surface of the carrier is from 10 to 1000 $\mu$m.

16. A process as claimed in any of claims 1 to 14, wherein the coat thickness of the catalytically active oxide material applied to the surface of the carrier is from 100 to 500 $\mu$m.

17. A process for the preparation of a catalyst which consists of a carrier and a catalytically active oxide material applied to the surface of the carrier, wherein a process as claimed in any of claims 1 to 16 is repeated periodically, ie. the carrier provided with the basecoat forms the carrier to be moistened in the subsequent period and then to be coated by contact with dry, finely divided, oxidic active material, etc.

18. A process as claimed in claim 17, wherein the carriers to be coated are introduced into a rotating container which passes the carriers periodically through two successive metering apparatuses, the first of which sprays the liquid binder onto the carriers rolling in the rotating container and the second of which meters in the catalytically active oxide material in finely divided form.

19. A process as claimed in any of claims 1 to 18, wherein the catalytically active oxide material is a multimetal oxide containing Mo and V.

20. A process as claimed in any of claims 1 to 18, wherein the catalytically active oxide material is a multimetal oxide containing Mo, Fe and Bi.

21. A coated catalyst consisting of a hollow cylindrical carrier having a length of from 2 to 10 mm, an external diameter from 4 to 10 mm and a wall thickness of from 1 to 4 mm and a catalytically active oxide material which is applied to the outer surface of the carrier and is of the formula I

$$Mo_{12}V_aX^1_bX^2_cX^3_dX^4_eX^5_fX^6_gO_n \qquad (I),$$

where

$X^1$     is W, Nb, Ta, Cr or Ce,
$X^2$     is Cu, Ni, Co, Fe, Mn or Zn,
$X^3$     is Sb or Bi,
$X^4$     is at least one or more alkali metals,
$X^5$     is at least one or more alkaline earth metals,
$X^6$     is Si, Al, Ti or Zr,
a     is from 1 to 6,
b     is from 0.2 to 4,
c     is from 0.5 to 18,
d     is from 0 to 40,
e     is from 0 to 2,
f     is from 0 to 4,
g     is from 0 to 40 and
n     is a number which is determined by the valency and frequency of the elements in I which differ from oxygen,

the applied catalytically active oxide material

- being applied in a coat thickness of from 10 to 1000 $\mu$m,

- and having a specific, catalytically active surface area of from 20 to 30 $m^2$/g and

- an abrasion of < 10, preferably < 5, particularly preferably < 0.5, % by weight in the turntable abrasion test.

22. A process for the preparation of acrylic acid by gas-phase catalytic oxidation of acrolein, wherein the catalyst used is a coated catalyst as claimed in claim 21.

23. A coated catalyst consisting of a hollow cylindrical carrier having a length from 2 to 10 mm, an external diameter from 4 to 10 mm and a wall thickness of from 1 to 4 mm and a catalytically active oxide material which is applied to the outer surface of the carrier and is of the formula II

$$Mo_{12}Bi_aFe_bX^1_cX^2_dX^3_eX^4_fO_n \qquad (II),$$

where

$X^1$     is nickel or cobalt,
$X^2$     is thallium, an alkali metal or an alkaline earth metal,
$X^3$     is phosphorus, arsenic, boron, antimony, tin, cerium, lead, niobium or tungsten,

$X^4$      is silicon, aluminum, titanium or zirconium,

a      is from 0.5 to 5,

b      is from 0.01 to 3,

c      is from 3 to 10,

d      is from 0.02 to 2,

e      is from 0 to 5,

f      is from 0 to 10 and

n      is a number which is determined by the valency and frequency of the elements in II which differ from oxygen,

the applied catalytically active oxide material

- being applied in a coat thickness of from 10 to 1000 $\mu$m,

- and having a specific catalytic surface area of from 20 to 30 $m^2$/g and

- an abrasion of < 10, preferably < 5, particularly preferably < 0.5, % by weight in the turntable abrasion test.

24. A process for the preparation of acrolein or methacrolein by gas-phase catalytic oxidation of propene or tert-butanol or isobutane or isobutene or tert-butyl methyl ether, wherein the catalyst used is a coated catalyst as claimed in claim 23.

25. A catalyst gas-phase oxidation process, wherein the catalyst used is the product of a process as claimed in any of claims 1 to 20.

26. A coated catalyst consisting of a carrier and a catalytically active oxide material applied to the surface of the carrier, obtainable by a process as claimed in any of claims 1 to 20.

**Revendications**

1. Procédé pour la préparation d'un catalyseur, qui est constitué d'un corps de support et d'une matière catalytiquement active de type oxyde, appliquée sur la surface du corps de support, dans lequel on humecte d'abord le corps de support avec un liant liquide, puis on fait adhérer à la surface du corps de support humecté une couche de matière active de type oxyde, par mise en contact avec une matière active de type oxyde sèche, finement divisée, et ensuite on élimine le liant liquide du corps de support humecté, revêtu avec la matière active de type oxyde, caractérisé en ce que l'on utilise en tant que liant liquide une solution constituée de 20 à 90 % en poids d'eau et de 10 à 80 % en poids d'un composé organique, dont le point d'ébullition ou la température de sublimation, sous la pression normale (1 atmosphère), est > 100°C, à l'exception de la préparation de catalyseurs dans lesquels on utilise pour la préparation

     a) en tant que support, des granules d'oxyde d'aluminium ayant un diamètre de 2 à 4 mm,
     b) en tant que liant liquide, une solution aqueuse d'acide lactique à 80 % en poids, et
     c) en tant que matière active de type oxyde, un oxyde de Cu-Cr-Al dopé au baryum.

2. Procédé selon la revendication 1, caractérisé en ce que la proportion du composé organique dans le liant liquide va de 10 à 50 % en poids.

3. Procédé selon la revendication 1, caractérisé en ce que la proportion du composé organique dans le liant liquide va de 20 à 30 % en poids.

4. Procédé selon l'une des revendications 1 à 3, caractérisé en ce que le composé organique est au moins un composé choisi dans l'ensemble comprenant des alcools mono- ou polyhydroxylés, des acides mono- ou polycarboxyliques organiques, des aminoalcools, des amides organiques mono- ou polyfonctionnels, des monosaccharides et des oligosaccharides.

5. Procédé selon l'une des revendications 1 à 4, caractérisé en ce que le composé organique est au moins un composé choisi dans l'ensemble comprenant l'éthylèneglycol, le 1,4-butanediol, le 1,6-hexanediol, le glycérol, l'acide propionique, l'acide oxalique, l'acide malonique, l'acide glutarique, l'acide maléique, l'éthanolamine, la diéthanolamine, le formamide, le glucose, le fructose, le saccharose et le lactose.

6. Procédé selon l'une des revendications 1 à 5, caractérisé en ce que le point d'ébullition du liant liquide sous la pression normale (1 atmosphère) est > 150°C.

7. Procédé selon l'une des revendications 1 à 6, caractérisé en ce que le corps de support est constitué d'oxyde d'aluminium, de silice, d'argile, de kaolin, de stéatite, de ponce, de silicate d'aluminium, de silicate de magnésium, de carbure de silicium, de dioxyde de zirconium ou de dioxyde de thorium.

8. Procédé selon l'une des revendications 1 à 7, caractérisé en ce que la rugosité superficielle $R_z$ du corps de support va de 40 à 100 $\mu$m.

9. Procédé selon l'une des revendications 1 à 8, caractérisé en ce que le volume total des pores du corps de support, par rapport au volume du corps de support, est $\leq$ 1 % en volume.

10. Procédé selon l'une des revendications 1 à 9, caractérisé en ce que l'extension maximale du corps de support va de 1 à 10 mm.

11. Procédé selon l'une des revendications 1 à 10, caractérisé en ce que l'on utilise comme corps de support des billes.

12. Procédé selon l'une des revendications 1 à 10, caractérisé en ce que l'on utilise comme corps de support des cylindres creux.

13. Procédé selon la revendication 12, caractérisé en ce que les cylindres creux ont une longueur de 2 à 10 mm, un diamètre externe de 4 à 10 mm et une épaisseur de paroi de 1 à 4 mm.

14. Procédé selon la revendication 12, caractérisé en ce que les cylindres creux ont une longueur de 3 à 6 mm, un diamètre externe de 4 à 8 mm et une épaisseur de paroi de 1 à 2 mm.

15. Procédé selon l'une des revendications 1 à 14, caractérisé en ce que l'épaisseur de couche de la matière catalytiquement active de type oxyde, appliquée sur la surface du corps de support, va de 10 à 1 000 $\mu$m.

16. Procédé selon l'une des revendications 1 à 14, caractérisé en se que l'épaisseur de couche de la matière catalytiquement active de type oxyde, appliquée sur la surface du corps de support, va de 100 à 500 $\mu$m.

17. Procédé pour la préparation d'un catalyseur qui est constitué d'un corps de support et d'une matière catalytiquement active de type oxyde, appliquée sur la surface du corps de support, caractérisé en ce que l'on utilise, répété de façon périodique, le procédé selon l'une des revendications 1 à 16, c'est-à-dire que le corps de support revêtu de base constitue dans la période suivante le "corps de support" à humecter et ensuite à revêtir par contact avec de la matière active de type oxyde sèche, finement divisée, etc.

18. Procédé selon la revendication 17, caractérisé en ce que les corps de support à revêtir sont introduits dans un récipient rotatif en rotation, qui fait passer les corps de support périodiquement sous deux dispositifs doseurs placés l'un à la suite de l'autre, dont le premier pulvérise le liant liquide sur les corps de support tournant dans le récipient rotatif en rotation et le second ajoute de façon réglée la matière catalytiquement active de type oxyde, sous forme finement divisée.

19. Procédé selon l'une des revendications 1 à 18, caractérisé en ce que la matière catalytiquement active de type oxyde est un oxyde multimétallique contenant Mo et V.

20. Procédé selon l'une des revendications 1 à 18, caractérisé en ce que la matière catalytiquement active de type oxyde est un oxyde multimétallique contenant Mo, Fe et Bi.

21. Catalyseur en forme de capsule, constitué d'un corps de support en forme de cylindre creux, ayant une longueur de 2 à 10 mm, un diamètre externe de 4 à 10 mm et une épaisseur de paroi de 1 à 4 mm, ainsi que d'une matière catalytiquement active de type oxyde, appliquée sur la surface externe du corps de support, de formule générale I

$$Mo_{12}V_a X_b^1 X_c^2 X_d^3 X_e^4 X_f^5 X_g^6 O_n \qquad (I)$$

dans laquelle les variables ont les significations suivantes:

$X^1$ représente W, Nb, Ta, Cr et/ou Ce,
$X^2$ représente Cu, Ni, Co, Fe, Mn et/ou Zn,
$X^3$ représente Sb et/ou Bi,
$X^4$ représente au moins un ou plusieurs métaux alcalins,
$X^5$ représente au moins un ou plusieurs métaux alcalinoterreux,
$X^6$ représente Si, Al, Ti et/ou Zr,
a va de 1 à 6,
b va de 0,2 à 4,
c va de 0,5 à 18,
d va de 0 à 40,
e va de 0 à 2,
f va de 0 à 4,
g va de 0 à 40 et
n est un nombre qui est déterminé par la valence et la fréquence des éléments différents de l'oxygène dans I,

la matière catalytiquement active de type oxyde qui est appliquée

- étant appliquée en une épaisseur de couche de 10 à 1 000 $\mu$m,
- ayant une surface spécifique, catalytiquement active, de 20 à 30 $m^2$/g et
- présentant, dans l'essai d'abrasion sur plateau tournant, une abrasion < 10 % en poids, de préférence < 5 % en poids et de façon particulièrement préférée < 0,5 % en poids.

22. Procédé pour la production d'acide acrylique par oxydation catalytique d'acroléine en phase gazeuse, caractérisé en ce que l'on utilise en même temps comme catalyseur un catalyseur en forme de capsule selon la revendication 21.

23. Catalyseur en forme de capsule, constitué d'un corps de support en forme de cylindre creux, ayant une longueur de 2 à 10 mm, un diamètre externe de 4 à 10 mm et une épaisseur de paroi de 1 à 4 mm, ainsi que d'une matière catalytiquement active de type oxyde, appliquée sur la surface externe du corps de support, de formule générale II

$$Mo_{12}Bi_aFe_bX^1_cX^2_dX^3_eX^4_fO_n \qquad\qquad (II)$$

dans laquelle les variables ont les significations suivantes:

$X^1$ représente le nickel et/ou le cobalt,
$X^2$ représente le thallium, un métal alcalin et/ou un métal alcalino-terreux,
$X^3$ représente le phosphore, l'arsenic, le bore, l'antimoine, l'étain, le cérium, le plomb, le niobium et/ou le tungstène,
$X^4$ représente le silicium, l'aluminium, le titane et/ou le zirconium,
a va de 0,5 à 5,
b va de 0,01 à 3,
c va de 3 à 10,
d va de 0,02 à 2,
e va de 0 à 5,
f va de 0 à 10 et
n est un nombre qui est déterminé par la valence et la fréquence des éléments différents de l'oxygène dans II,

la matière catalytiquement active de type oxyde qui est appliquée

- étant appliquée en une épaisseur de couche de 10 à 1 000 $\mu$m,
- ayant une surface spécifique catalytique de 20 à 30 $m^2$/g et
- présentant, dans l'essai d'abrasion sur plateau tournant, une abrasion < 10 % en poids, de préférence < 5 % en poids et de façon particulièrement préférée < 0,5 % en poids.

24. Procédé pour la production d'acroléine ou de méthacroléine par oxydation catalytique en phase gazeuse du propène ou du tert-butanol ou de l'isobutane ou de l'isobutène ou de l'éther tert-butylméthylique, caractérisé en ce que l'on utilise en même temps comme catalyseur un catalyseur en forme de capsule selon la revendication 23.

25. Procédé d'oxydation catalytique en phase gazeuse, caractérisé en ce que l'on utilise comme catalyseur le produit d'un procédé selon l'une des revendications 1 à 20.

26. Catalyseur en forme de capsule, constitué d'un corps de support et d'une matière catalytiquement active de type oxyde, appliquée sur la surface du corps de support, pouvant être obtenu par un procédé selon l'une des revendications 1 à 20.